(19) **Europäisches Patentamt**

**European Patent Office**

**Office européen des brevets**

(11) **EP 0 695 217 B2**

(12) **NOUVEAU FASCICULE DE BREVET EUROPEEN**

(45) Date de publication et mention de la décision concernant l'opposition:
**14.03.2001 Bulletin 2001/11**

(45) Mention de la délivrance du brevet:
**03.09.1997 Bulletin 1997/36**

(21) Numéro de dépôt: **94913674.1**

(22) Date de dépôt: **21.04.1994**

(51) Int Cl.⁷: $B01J\ 21/06$, $B01J\ 37/34$, $C07D\ 301/12$, $C07D\ 301/19$

(86) Numéro de dépôt international:
**PCT/FR94/00450**

(87) Numéro de publication internationale:
**WO 94/23834 (27.10.1994 Gazette 1994/24)**

(54) **CATALYSEUR A BASE DE SILICE ET DE TITANE ET SON UTILISATION DANS L'EPOXYDATION D'OLEFINES**

KATALYSATOR AUF BASIS VON SILITIUMDIOXID UND TITAN UND SEINE ANWENDUNG FÜR DIE EPOXIDIERUNG VON OLEFINEN

CATALYST BASED ON SILICA AND TITANIUM, AND UTILISATION OF SAID CATALYST FOR THE EPOXYDATION OF OLEFINS

(84) Etats contractants désignés:
**DE FR**

(30) Priorité: **22.04.1993 FR 9304752**

(43) Date de publication de la demande:
**07.02.1996 Bulletin 1996/06**

(73) Titulaire: **Atofina**
**92800 Puteaux (FR)**

(72) Inventeurs:
- **TEISSIER, Rémy**
  **F-69340 Francheville (FR)**
- **KERVENNAL, Jacques**
  **F-69005 Lyon (FR)**

(56) Documents cités:
| | |
|---|---|
| **EP-A- 0 129 814** | **EP-A- 0 345 856** |
| **EP-A- 0 347 926** | **GB-A- 1 332 526** |
| **US-A- 3 285 898** | **US-A- 3 923 843** |
| **US-A- 4 021 454** | **US-A- 4 410 501** |

**Description**

La présente invention concerne les catalyseurs solides à base de silice particulaire comportant du titane et leurs utilisations.

Ces catalyseurs sont utilisés dans un vaste domaine de réactions chimiques d'oxydation, notamment l'époxydation d'oléfines par le peroxyde d'hydrogène ou les hydroperoxydes organiques, l'ammoximation de cétones et l'hydroxylation du phénol.

Ces catalyseurs ont fait l'objet de nombreuses publications. Ainsi le brevet US 5,082,641 décrit un procédé de synthèse de zéolites MFI contenant du titane.

Le brevet Us 4,968,842 décrit un procédé catalytique de synthèse d'oximes. Le catalyseur peut être préparé par imprégnation d'une silice amorphe commerciale ayant une grande surface spécifique (par exemple un produit micro sphéroïdal) et un volume poreux élevé, à l'aide de solutions aqueuses ou non aqueuses de composés solubles du titane.

Comme sources de titane soluble sont citées les alkyltitanates, les halogénures de titane, en particulier le tétrachlorure de titane et trichlorure de titane, les titanates complexes et notamment l'ammonium hexafluorotitanate.

L'exemple 3 de ce brevet décrit la préparation d'un catalyseur comportant l'imprégnation d'une silice amorphe par une solution de tétrachlorure de titane dans de l'acide chlorhydrique 6 M.

La silice ainsi imprégnée est séchée à 120°C puis calcinée à 200°C. Le catalyseur ainsi obtenu est décrit comme contenant 8,1% en poids de titane exprimé en $TiO_2$. Ce catalyseur peut être utilisé dans une réaction d'ammoximation ou il peut être calciné à des températures de 50 à 800°C.

Le brevet Us 4,021,454 décrit, dans son exemple la préparation d'un catalyseur à base de silice contenant du titane. Ce catalyseur est préparé en mélangeant dans du n-heptane une silice pyrogénique commerciale, ayant une surface spécifique de 390 $m^2$/g, avec du tétrachlorure de titane, à une température de 97°C. L'eau formée est éliminée en recueillant l'azéotrope eau-heptane. L'acide chlorhydrique (HCI) formé est entraîné par un courant d'azote. Le mélange réactionnel est ensuite additionné d'eau pour initier le dégagement de HCI supplémentaire lors d'un chauffage à 97°C pendant 16 heures. Le mélange est ensuite évaporé et le résidu est séché sous pression réduite à 120°C. Le catalyseur obtenu à base de silice, contient 4% en poids de titane. Ce catalyseur est utilisé dans l'époxydation du chlorure d'allyle ou de l'alcool allylique par l'hydroperoxyde de t-Butyle.

Le document "Characterisation and catalytic properties of titanium incorporated into zeolites and silicas from titanium trifluoride, P.J. Kooyman, J.C. Jansen and H. Van Bekkum, 9th International Zeolite Conference, Montreal July 1992", décrit la préparation de catalyseurs par imprégnation de silices amorphes par du trifluorure de titane en milieux aqueux. Une solution A est préparée en dissolvant du trifluorure de titane dans de l'eau contenant de l'acide fluorhydrique, puis la silice amorphe est chauffée en autoclave à 180°C pendant 90 minutes avec une portion de solution A diluée par de l'eau.

Après refroidissement le solide est recueilli, lavé à l'eau et enfin séché à 120°C pour donner le catalyseur. Celui ci est utilisé dans l'hydroxylation du phénol par le peroxyde d'hydrogène.

Le brevet Us 4,410,501 décrit la préparation d'un catalyseur solide à base de silice comportant du titane, connu sous le nom de titanosilicalite ou TS-1 et possédant une structure cristallisée de zéolite.

Le document "La Chimica & Industria 72,1990, pp. 598-603, Paolo ROFFIA et Coll. "A new process for cyclohexanonoxime" rapporte la structure du catalyseur TS-1 préparé selon US 4,410,501 et calciné à 500°C. D'après ces auteurs la TS-1 diffère de la silicalite, par le remplacement de quelques atomes de silicium par des atomes de titane (fig. 2):

"L'insertion d'atomes de titane dans le réseau cristallin de la zéolite conduit à une structure dans laquelle :

- les atomes de titane sont isolés les uns des autres,
- la plupart d'entre eux peuvent être considérés comme des atomes de surface,
- ils prennent une coordination tétraédrique,
- ils sont capables de coordonner deux ligands pour prendre la configuration d'une coordination octaédrique, typique des composés de titane".

Ces auteurs ont préparés également des échantillons de titane supporté par de la silice en imprégnant une silice commerciale, amorphe et microspheroïdale (420 $m^2$/g) avec du di-isopropyl-bistriethyl-éthanolamine-titanate en solution, puis calcination à 200°C. Dans des exemples comparatifs indiqués en tableau 4, ces auteurs montrent que le catalyseur TS-1 donne de bien meilleurs résultats en terme de conversion et de sélectivité que les catalyseurs amorphes, dans la réaction d'ammoximation de la cyclohéxanone par $NH_3$/$H_2O_2$ dans un mélange eau/t-butanol,

Ces auteurs expliquent cette moins bonne activité par le fait que la technique de support ou d'ancrage du titane tétravalent à une silice amorphe ne peut probablement pas fixer fermement dans une structure tétraédrique des atomes isolés de titane à la surface même de la silice, mais au contraire produit ou conduit rapidement à la formation d'amas de dioxyde de titane, à la surface, dans une coordination octaédrique même à de faibles concentrations en titane. Cette agrégation d'atomes de titane ne serait pas aussi active que les atomes de titane isolés dans la structure cristalline de la TS-1.

A. Zecchina et coll. ont publié dans "Zeolite Chemistry and Catalysis", P.A. Jacobs et al., 1991, pp. 251-25; Elsevier Science Publishers B.V, Amsterdam, une étude intitulée : "Framework and extraframework Ti in titanium-silicalite : investigation by means of physical methods".

Ces auteurs décrivent une méthode d'analyse, par une spectroscopie de réflexion UV-Visible, permettant de distinguer le titane dans le réseau, du titane hors réseau cristallin.

Selon eux, les atomes de titane (IV) isolés dans le réseau dans une coordination tétraédrique correspondent à une bande de 48000 cm$^{-1}$ (208 nm) ; les atomes du titane (IV) isolés dans un environnement octaédrique correspondent à une bande à 42000 cm$^{-1}$ (238 nm) ; les atomes de Ti (IV) non-isolés, dans une coordination octaédrique et formant des amas d'oxydes, absorbent à une fréquence plus basse. Par exemple, avec l'Anatase ($TiO_2$) on observe une absorption à 30500 cm$^{-1}$ (328 nm).

D'après ces auteurs, il serait possible de détecter la présence d' environ 0,03% en poids d'Anatase dans des mélanges de silicalite (sans Ti) et d'anatase.

La demanderesse a vérifié, en utilisant cette technique de spectrographie U.V., que les catalyseurs à base de silice amorphe imprégnée par des sels de titane qui ont été décrits jusqu'à maintenant contenaient une forte proportion en poids d'amas de $TiO_2$.

Ainsi l'exemple 1 de US 4,021,454 décrivant l'imprégnation d'une silice amorphe par TiCl$_4$, conduit à une silice amorphe contenant des amas de $TiO_2$.

L'exemple 3 de US 4,968,842 décrivant l'imprégnation d'une silice amorphe par TiCl$_4$ dans une solution de HCl conduit également à une silice amorphe contenant une forte proportion d'amas de $TiO_2$.

Les silices amorphes de P.J. Kooyman (voir ci-dessus) imprégnées par TiF$_3$ donnent également des silices amorphes contenant essentiellement des amas de $TiO_2$.

Malgré l'enseignement de Paolo ROFFIA et coll. (voir ci-dessus) qui dissuade d'imprégner une silice amorphe car conduisant rapidement à la formation d'amas de dioxyde de titane à la surface de cette silice, la demanderesse s'est fixée le but d'obtenir des silices amorphes contenant essentiellement des atomes de titane isolés dans la silice avec une teneur en poids beaucoup plus faible en amas de $TiO_2$ que les silices amorphes contenant du titane connues à ce jour et même d'obtenir des silices amorphes au titane dans lesquelles le $TiO_2$ en amas ne serait pas détectable par la méthode U.V-Vis. de A. Zecchina et coll. En effet, il est connu que $TiO_2$ en amas catalyse la décomposition du peroxyde d'hydrogène.

D'une manière inattendue il a été maintenant trouvé que les buts ci-dessus pouvaient être atteints en imprégnant dans certaines conditions expérimentales des silices amorphes par du trifluorure de titane ou du tétrafluorure de titane. Le procédé d'obtention des catalyseurs selon la présente invention comporte la préparation d'un mélange réactionnel contenant un liquide non anhydre, une silice particulaire amorphe initiale, du TiF$_3$ ou TiF$_4$ en une quantité de 0,1 à 10% en poids de titane (Ti) par rapport au poids de la silice initiale ; on porte le mélange à une température choisie de 20°C à la température de reflux dudit liquide sous pression atmosphérique et on le laisse réagir jusqu'à ce que la silice initiale ait absorbé une quantité choisie de titane comprise entre les 0,1 et 10%, pour donner ainsi le catalyseur solide en suspension dans le liquide.

Avantageusement, le mélange n'est pas additionné d'HCl ou de HF

Le liquide du milieu réactionnel peut être de l'eau ou un composé organique inerte à l'action de TiF$_4$ ou TiF$_3$. Ce composé est choisi de préférence parmi les éthers, les cétones, les esters et les alcools.

La température du milieu réactionnel est de 20°C à environ 200°C. Lorsque le liquide est de l'eau, la température est de 20°C à environ 100°C. Lorsque le liquide est un composé organique, la température est de 20°C à environ 200°C.

Toute silice amorphe à grande surface spécifique peut servir de silice initiale. Avantageusement cette silice est obtenue par précipitation et sa surface spécifique est comprise en 200 m$^2$/g et 500 m$^2$/g.

De préférence, la silice initiale a une teneur en fer, nickel, chrome ou tout autre métal de transition, inférieure à 200 p.p.m. ou mieux encore inférieure à 100 p.p.m. En effet ces métaux peuvent entraîner la décomposition du peroxyde d'hydrogène selon une réaction du type Fenton.

On préfère le plus souvent séparer le catalyseur solide obtenu dudit liquide, notamment par filtration. Le catalyseur solide ainsi séparé peut être soumis à un séchage. De préférence, le catalyseur solide séparé et éventuellement séché est soumis à une calcination pratiquée à une température de 200 à 700°C.

Il est possible également de soumettre le catalyseur solide séparé, éventuellement séché et/ou calciné, à un lavage par une solution aqueuse basique afin de diminuer la teneur en élément fluor dudit catalyseur.

Avantageusement, ce catalyseur ainsi lavé est séché puis soumis à une calcination pratiquée à une température de 200° à 700°C.

De préférence, le catalyseur séparé du liquide, séché, puis calciné, est traité par du peroxyde d'hydrogène aqueux, puis filtré et enfin séché. Le catalyseur ainsi obtenu a une teneur faible en fluor et de plus l'avantage de garder une teneur sensiblement constante en titane lors de son utilisation prolongée et/ou recyclée en époxydation.

Les catalyseurs obtenus peuvent être définis chacun comme un catalyseur solide à base de silice particulaire amorphe comportant du titane (IV), et montrant en spectrographie UV-Vis, de réflexion une bande d'absorption à environ 240 nm, avec le titane présent dans le catalyseur en une quantité en poids comprise entre 0,1 et 10% par rapport au poids de la silice et un rapport r de l'intensité d'absorption mesurée à 240 nm sur l'intensité d'absorption mesure à 330 nm égal ou supérieur à 3.

L'intensité d'absorption à 240 nm varie dans le même sens que l'accroissement de la teneur pondérale en Ti (IV) isolé dans la silice alors que l'intensité d'absorption à 330 nm varie dans le même sens que la teneur pondérale des amas distincts de $TiO_2$ sur la silice.

Ainsi plus le rapport r est grand, plus le catalyseur contient de titane isolé par rapport en titane du type amas de $TiO_2$ et ceci pour une même quantité globale de titane.

Avantageusement r est égal ou supérieur à 4. De préférence, r est égal ou supérieur à 8.

Les catalyseurs de la présente invention peuvent être utilisés pour époxyder une oléfine par du peroxyde d'hydrogène ou un hydroperoxyde organique notamment l'hydroperoxyde de tertiobutyle.

En plus de la description qui précède, l'invention sera mieux comprise à l'aide des exemples qui vont suivre et qui sont donnés à titre illustratif et non limitatif.

Dans ces exemples :

Le $TiF_4$ est commercial, en poudre, d'une pureté de 98% en provenance de chez STREM sous la référence 93-2222.

Le $TiF_3$ est de STREM sous la référence 93-2224.

La préparation du catalyseur se fait généralement en mettant en suspension dans un liquide la quantité nécessaire de $TiF_3$ ou de $TiF_4$, puis on porte la suspension à une température de 40°C à 120°C. On introduit alors la silice particulaire amorphe et on laisse agiter le milieu à la même température pendant une durée variant de 15 minutes à 6 heures, de préférence 30 minutes à 2 heures. On laisse refroidir et on filtre. On sèche le solide en étuve (8 heures à 120°C sous une pression de $1,33 \times 10^2$.Pa quand le liquide utilisé est le diglyme).

Après séchage, on préfère pour augmenter la sélectivité en époxyde et en diol du catalyseur, le calciner sous air à une température comprise entre 200°C et 700°C pendant quelques heures, de préférence entre 350°C et 500°C pendant deux à trois heures.

Le dosage chimique du titane est effectué de manière classique par minéralisation et absorption atomique.

Le dosage chimique du fluor est effectué de manière usuelle par minéralisation, décomplexation et utilisation d'une électrode spécifique commerciale.

La spectroscopie UV-Vis de réflexion est effectuée selon la publication ci-dessus de A. Zecchina et al., à l'aide d'un appareil Perkin Elmer Lambda 9 par rapport à une référence constituée par du sulfate de magnésium.

L'intensité d'absorption en reflexion diffuse est égale à la fonction f(R) définie selon la formule de Kubela-Munk:

$$f(R)= (1-R^2)/2R,$$

R étant la réflectance mesurée.

L'activité catalytique des différents catalyseurs est déterminée par une époxydation d'une oléfine réalisée dans des conditions analogues à celles d'une époxydation par le peroxyde d'hydrogène en catalyse homogène. L'oléfine utilisée est le cyclohexène qui ne peut être époxydé en présence des titanosilicalites comme la TS-1.

La quantité de catalyseur solide testé est comprise entre 0,5 et 10% en poids par rapport au poids du mélange réactionnel. De préférence cette quantité est de 2 à 5% en poids.

Le peroxyde d'hydrogène est introduit sous la forme d'une solution aqueuse à 70% en poids

Le solvant est choisi parmi les solvants classiques utilisés dans l'époxydation par $H_2O_2$, tels que le diglyme ou le tertiobutanol.

Chaque époxydation a été menée dans un réacteur fermé sur cuve d'eau, afin de déterminer le volume de gaz dégagé et ainsi le taux de décomposition de $H_2O_2$ en $H_2O$ et $O_2$. L'obtention de 10 ml de $O_2$ gazeux dans les conditions normales de température (20°) et de pression correspond à environ 1 mmole de $H_2O_2$ décomposé.

**Préparation des catalyseurs :**

Exemple 1

Dans un réacteur agité, on introduit 52 g de diglyme, 0,2 g de $TiF_4$ solide. En agitant on porte ce mélange à 60°C et on introduit 5 g de silice particulaire amorphe (Degussa FK 310) ayant une surface spécifique de 400 m$^2$/g.

On laisse sous agitation à 60°C pendant une heure, puis on laisse refroidir et on filtre. On détermine dans le filtrat la concentration en titane et fluor. Le filtrat contient 200 p.p.m. de F et 15 p.p.m. de Ti.

Le solide est séché à 120°C sous 1,33. 10$^2$.Pa, pendant 8 heures. On dose la teneur en fluor et en titane du catalyseur. Il contient en poids/poids 1,9% de F et 1,25% de Ti.

Le rapport r = 10

Exemple 2

Cet exemple est réalisé de manière identique à l'exemple 1 mais après le séchage on calcine à 350°C pendant trois heures.

Exemple 3

Cet exemple est réalisé de manière identique à l'exemple 1 mais après le séchage on calcine à 500°C pendant cinq heures. Le rapport r est égal à 8.

On dose chimiquement le fluor et le titane:

Ti = 1,45%
F = 2,2%

Exemple 4

Identique à l'exemple 1, mais on ne filtre pas. Le catalyseur, en suspension dans le diglyme, est utilisé tel quel.

Exemple 5

Variante de l'exemple 1 par le fait qu'on introduit simultanément la silice et TiF$_4$ dans le diglyme à température ambiante, puis on porte le mélange à 60°C pendant 1 heure.

Exemple 6

Identique à l'exemple 1 sauf que l'on remplace le diglyme par la même masse de Dioxane.

Exemple 7

Identique à l'exemple 1, sauf que l'on remplace le diglyme par la même masse d'acétate d'éthyle.

Exemple 8

Identique à l'exemple 1, sauf que l'on remplace le diglyme par la même mesure d'acétone.

Exemple 9

Dans 250g de diglyme, on dissout 1g de TiF$_4$ à 60°C, sous agitation. Puis on ajoute 25g de silice Degussa FK 310 et on laisse réagir une heure à 60°C sous agitation. On filtre le solide et on la sèche sous une pression de 1,33.10$^2$.Pa , pendant 14 heures.

Exemple 10

Une partie aliquote de 5 g de solide obtenu de l'exemple 9 est calcinée à 500°C pendant 3 heures.

Exemple 11

12,5g du solide obtenu de l'exemple 9 est mis en suspension dans 500 ml d'une solution aqueuse à 5g/l de carbonate d'ammonium, dont le pH initial est de 8,5. Le traitement est poursuivi deux à trois heures environ jusqu'à stabilisation du pH à 7,1 environ. Puis on filtre et on sèche durant 14 heures à 120°C sous 1,33.10$^2$.Pa.

Le solide contient 0,3% poids/poids de fluor au lieu des 1,9% initiaux.

Exemple 12

Identique à l'exemple 11, mais le solide séché est soumis à une calcination à 500°C pendant 3 heures.

Exemple 13

Identique à l'exemple 10 mais le solide calciné est ensuite traité comme à l'exemple 11.

Exemple 14

Identique à l'exemple 13 mais le solide obtenu est finalement soumis de nouveau à une calcination à 500°C pendant 3 heures.

Exemple 15

Dans un réacteur agité, on introduit 50g d'eau désionisée, 0,2g de $TiF_4$ solide. En agitant, on porte ce mélange à 60°C et on introduit 5g de silice Degussa FK 310 ayant comme surface spécifique 400 $m^2$/g. On laisse sous agitation à 60°C pendant une heure.
On filtre le solide puis on le sèche durant 16 heures à 120°C sous 0,66 . $10^2$ Pa.

Exemple 16

Identique à l'exemple 15 mais après le séchage, on calcine le solide sous air à 500°C pendant 3 heures.

Exemple 17

Dans un réacteur agité, on introduit 50g de tertiobutanol et 0,2g de $TiF_4$. En agitant, on porte ce mélange à 60°C et on introduit 5g de silice Degussa FK 310 ayant une surface spécifique de 400 $m^2$/g. On laisse sous agitation à 60°C pendant une heure. On filtre le solide que l'on sèche durant 16 heures à 120°C sous 0,66.$10^2$ Pa.

Exemple 18

Identique à l'exemple 1, sauf que les 0,2g de $TiF_4$ solide sont remplacés par 0,2g de $TiF_3$.

Exemple 19

Identique à l'exemple 18, mais après le chauffage le solide est calciné à 500°C pendant 3 heures.

Exemple 20

Identique à l'exemple 18, mais le diglyme est remplacé par de l'eau.

Exemple 21

On mélange à la température de 20°C, 0,2g de $TiF_4$ dans 50g d'eau désionisée et 5g de silice amorphe Degussa FK 310. Au bout d'une heure d'agitation à 20°C, on filtre et sèche le solide recueilli pendant 16 heures à 120°C sous 40.$10^2$ Pa, puis on le calcine trois heures à 350°C sous l'air ambiant.

Exemple comparatif 22

L'exemple 3 du brevet US 4,968,842 a été reproduit en utilisant de la silice Degussa FK 310.

Exemple comparatif 23

L'exemple 1 du brevet US 4,021,454 a été reproduit en utilisant de la silice Degussa FK 310.

Exemple comparatif 24

La préparation, divulguée par l'article de P.J. Kooyman et al. cité ci-dessus, du catalyseur à base de silice amorphe a été reproduit mais en utilisant de la silice Degussa FK

Les différents solides obtenus dans les exemples 1 à 24 ont été testés comme catalyseur d'époxydation du cyclohexène par le peroxyde d'hydrogène ou l'hydroperoxyde de tertiobutyle selon le mode opératoire général suivant :

Exemple 25

Une partie du catalyseur obtenu dans l'exemple 3 est mise en suspension, pendant 1 heure, à la température de 20°C, dans 50 ml de peroxyde d'hydrogène aqueux à 10% en poids dans de l'eau. Le catalyseur ainsi traité, est filtré puis séché à 120°C pendant 8 heures sous un vide de $1,33.10^2$ Pa.

Le catalyseur ainsi obtenu contient 0,2% de fluor et 1% de titane. Le rapport r est de 8, inchangé par rapport au catalyseur de l'exemple 3.

Exemple 26

Le procédé de l'exemple 25 est répété de la même manière sur une partie du catalyseur obtenu par l'exemple 25.

Le catalyseur ainsi obtenu contient 0,1% de fluor et 0,9% de titane.

Epoxydafion par $H_2O_2$

Dans un réacteur agité, thermostaté, muni d'un condenseur à reflux, d'une prise de température, et d'un dispositif d'introduction d'un réactif liquide, on introduit la charge suivante :

- 52g de diglyme
- 82g de cydohexène (1 mole)
- 5g de catalyseur obtenu dans l'un des exemples 1 à 19 selon le tableau I ci après.

Le condenseur à reflux est relié à une cuve à eau par l'intermédiaire de tuyau et de dispositifs de sécurité anti-retour classiques.

On porte le milieu réactionnel sous agitation et à une température déterminée qui peut être celle du reflux à environ 94°C.

On introduit alors en 30 minutes 0,052 mole (1.26g) de $H_2O_2$ à 70% en poids dans l'eau en solution dans 20g de diglyme.

Après l'introduction, on laisse réagir à la température déterminée pendant une durée choisie entre une et quatre heures. On observe au niveau de la cuve à eau l'éventuel dégagement de gaz

On laisse refroidir. On vérifie par dosage qu'il ne reste plus de $H_2O_2$ dans le milieu réactionnel (oxydation de KI en milieu acide, puis dosage de l'iode par du thiosulfate de sodium (selon "G. Chariot - Analyse quantitative minérale p.80 - Masson 1955").

On dose les époxydes par le dosage classique au HCI en milieu éthanolique en présence de chlorure de magnésium (selon "Organic analysis I p. 127 - Interscience 1953").

On analyse le mélange réactionnel par chromatographie gazeuse (chromatographie H.P. 5590, colonne capillaire de diamètre 0,53 mm d'une longueur de 30 m, garnie de carbowax, le gaz vecteur était l'hélium. Le programme : 5 minutes à 70°C puis montée à 12°C/min. jusqu'à 240°C. L'étalonnage est externe). On mesure ainsi la teneur en oxyde de cyclohexène et la teneur en 1,2 - Cyclohexanediol.

Le tableau I montre les résultats obtenus, avec la conversion et les sélectivités par rapport au peroxyde d'hydrogène.

$$\text{Conversion en \%} = 100.([H_2O_2]_o - [H_2O_2]_f) / [H_2O_2]_o$$

$$\text{Sélectivité en époxy en \%} = 100.([\text{époxy}]/ ([H_2O_2]_o - [H_2O_2]_f)$$

$$\text{Sélectivité en diol en \%} = 100.([\text{diol-1,2}]/([H_2O_2]_o - [H_2O_2]_f)$$

Sélectivité totale = sélectivité en époxy + sélectivité en diol

$[H_2O_2]_o$ représente le nombre de moles de peroxyde d'hydrogène introduit dans le milieu réactionnel.

$[H_2O_2]_f$ représente le nombre de moles de peroxyde d'hydrogène restant dans le milieu en fin d'opération.

[époxy] représente le nombre de moles d'époxy dans le milieu, en fin d'opération.

Il ressort du tableau I, que les catalyseurs obtenus aux exemples 3 et 25 sont les meilleurs, car conduisant respectivement à une sélectivité totale de 96% pour un taux de conversion de 100% du peroxyde d'hydrogène, et à une sélectivité totale de 97% pour un taux de conversion de 90% du peroxyde d'hydrogène.
Par contre, les catalyseurs de l'état de la technique, obtenus selon les exemples comparatifs 22 à 24, décomposent fortement le peroxyde d'hydrogène et/ou entraînent une faible sélectivité totale.

Epoxydation par l'hydroperoxyde de tertiobutyle.

Dans un réacteur identique à celui utilisé ci-dessus pour l'époxydation au peroxyde d'hydrogène, on introduit :

- 2,6g du catalyseur obtenu à l'exemple 21
- 41g (0,5 mole) du cyclohexène
- 90g de tertiobutanol.

On chauffe ce mélange hétérogène au reflux qui débute à 75°C. On coule en une heure 0,026 moles d'hydroperoxyde de tertiobutyle dessous dans 10g de tertiobutanol. On laisse réagir 5 heures au reflux. Le dégagement gazeux est de 30 ml. Un dosage (KI, puis thiosulfate de sodium) montre qu'il reste 0,007 mole d'hydroperoxyde, soit une conversion de ce dernier de 73%. Par la chromatographie gazeuse on dose 9 ml d'oxyde de cyclohexène soit une sélectivité époxy de 47% et des traces de cyclohéxane diol -1,2.

## TABLEAU I

| Catalyseur de l'exemple N° | Température du milieu réactionnel en °C | durée de réaction en heures | Dégagement gazeux en ml | Taux de Conversion en % | Sélectivité époxy en % | Sélectivité diol -1,2 en % | Sélectivité totale époxy + diol-1,2 en % | Rapport r |
|---|---|---|---|---|---|---|---|---|
| 1 | 94 | 1 | 20 | 100 | 52 | 8 | 60 | 10 |
| 2 | 94 | 1 | 60 | 100 | 62 | 15 | 77 | 9 |
| 3 | 94 | 1 | 0 | 100 | 69 | 27 | 96 | 8 |
| 4 | 94 | 1 | 20 | 100 | 43 | 8 | 51 | – |
| 5 | 94 | 1 | 50 | 100 | 46 | 6 | 52 | 10 |
| 6 | 94 | 1 | 30 | 100 | 52 | 8 | 60 | 8 |
| 7 | 94 | 1 | 0 | 100 | 48 | 8 | 56 | 6 |
| 8 | 94 | 1 | 10 | 100 | 52 | 8 | 60 | 15 |
| 9 | 94 | 1 | 30 | 100 | 51 | 10 | 60 | 15 |
| 10 | 94 | 1 | 30 | 100 | 32 | 15 | 47 | 7 |
| 11 | 94 | 1 | 30 | 100 | 33 | 22 | 55 | 16 |
| 12 | 94 | 1 | 50 | 100 | 49 | 8 | 57 | 10 |
| 13 | 94 | 1 | 10 | 100 | 14 | 20 | 34 | 7 |
| 14 | 94 | 1 | 20 | 100 | 52 | 4 | 56 | 7 |
| 15 | 94 | 1 | 10 | 100 | 52 | 14 | 66 | 120 |
| 16 | 94 | 1 | 30 | 100 | 56 | 16 | 72 | 148 |
| 17 | 94 | 1 | 20 | 84 | 44 | 5 | 48 | 11 |
| 18 | 94 | 1 | 40 | 100 | 32 | 16 | 48 | 31,5 |
| 19 | 94 | 1 | 10 | 100 | 44 | 16 | 60 | 50 |
| 20 | 94 | 1 | 10 | 100 | 40 | 24 | 64 | 4,7 |
| 21 | 94 | 1 | – | – | – | – | – | 10 |
| 22* | 94 | 1 | 400 | 85 | 2 | 0 | 2 | 0,4 |
| 23* | 94 | 1 | 190 | 92 | 48 | 0 | 48 | 2,2 |
| 24* | 94 | 1 | 50 | 48 | 8 | 0 | 8 | 0,2 |
| 25 | 80 | 3,5 | 0 | 90 | 72 | 25 | 97 | 8 |

* Exemples comparatifs

## Revendications

1. Procédé d'obtention d'un catalyseur solide à base de silice particulaire amorphe comportant du titane (IV), et montrant en spectrographie ultraviolet de réflexion une bande d'absorption à environ 240 nm, et avec un rapport r de l'intensité d'absorption U.V. mesurée à 240 nm sur l'intensité d'absorption mesurée à 330 nm égal ou supérieur à 3, caractérisé en ce qu'il comporte la préparation d'un mélange réactionnel contenant un liquide non anhydre, une silice particulaire amorphe initiale, du $TiF_3$ ou $TiF_4$ en une quantité de 0,1 à 10 % en poids de titane (Ti) par rapport au poids de la silice initiale et que l'on porte ce mélange à une température choisie de 20°C à la température de reflux dudit liquide, sous la pression atmosphérique, et on le laisse réagir jusqu'à ce que la silice initiale ait absorbé une quantité choisie de titane comprise entre 0,1 et 10 % par rapport au poids de la silice, pour donner ainsi le catalyseur solide en suspension dans le liquide.

2. Procédé suivant la revendication 1 caractérisé en ce que le mélange n'est pas additionné d'HCl ou de HF.

3. Procédé suivant la revendication 1 ou 2 caractérisé en ce que le liquide est de l'eau.

4. Procédé suivant la revendication 1 ou 2 caractérisé en ce que le liquide est un composé organique inerte à l'action du $TiF_3$ ou du $TiF_4$.

5. Procédé suivant l'une des revendications 1 à 4 caractérisé en ce que ladite température est de 20°C à environ 200°C.

6. Procédé suivant l'une des revendications 1 à 5 caractérisé en ce que le catalyseur en suspension dans le liquide est filtré puis séché.

7. Procédé suivant la revendication 6, caractérisé en ce que le catalyseur séché est calciné à une température de 200°C à 700°C.

8. Procédé suivant la revendication 7 caractérisé en ce que le catalyseur calciné est traité par du peroxyde d'hydrogène, puis filtré et séché.

9. Utilisation d'un catalyseur obtenu suivant l'une des revendications 1 à 8, pour époxyder une oléfine par du peroxyde d'hydrogène ou un hydroperoxyde organique.

**Patentansprüche**

1. Verfahren zur Herstellung eines festen Katalysators auf der Basis einer amorphen, teilchenförmigen Kieselsäure, der Titan(IV) enthält, dessen Ultraviolett-Reflexions-spektrum bei etwa 240 nm eine Absorptionsbande zeigt, wobei das Verhältnis r der bei 240 nm gemessenen UV-Absorptionsintensität zu der bei 330 nm gemessenen Absorptionsintensität mindestens 3 beträgt, dadurch gekennzeichnet, daß ein Reaktionsgemisch hergestellt wird, das eine nicht wasserfreie Flüssigkeit, eine als Ausgangsmaterial eingesetzte amorphe, teilchenförmige Kieselsäure, $TiF_3$ oder $TiF_4$ in einer Menge von 0,1 bis 10 Gew.-% Titan (Ti), bezogen auf das Gewicht der als Ausgangsmaterial eingesetzten Kieselsäure, enthält, daß dieses Gemisch bei Atmosphärendruck auf eine Temperatur erwärmt wird, die im Bereich von 20 °C bis zur Rückflußtemperatur der Flüssigkeit liegt, und daß man das Gemisch reagieren läßt, bis die als Ausgangsmaterial eingesetzte Kieselsäure eine Menge an Titan absorbiert hat, die im Bereich von 0,1 bis 10 %, bezogen auf das Gewicht der Kieselsäure, liegt, um so eine Suspension aus dem festen Katalysator und der Flüssigkeit zu erhalten.

2. Verfahren nach Anspruch 1, dadurch gekennzeichnet, daß zu dem Gemisch kein HCl oder HF gegeben wird.

3. Verfahren nach Anspruch 1 oder 2, dadurch gekennzeichnet, daß es sich bei der Flüssigkeit um Wasser handelt.

4. Verfahren nach Anspruch 1 oder 2, dadurch gekennzeichnet, daß es sich bei der Flüssigkeit um eine organische Verbindung handelt, die gegenüber der Einwirkung von $TiF_3$ oder $TiF_4$ inert ist.

5. Verfahren nach einem der Ansprüche 1 bis 4, dadurch gekennzeichnet, daß die Temperatur im Bereich von 20 bis etwa 200 °C liegt.

6. Verfahren nach einem der Ansprüche 1 bis 5, dadurch gekennzeichnet, daß der in der Flüssigkeit suspendierte Katalysator abfiltriert und anschließend getrocknet wird.

**7.** Verfahren nach Anspruch 6, dadurch gekennzeichnet, daß der getrocknete Katalysator bei einer Temperatur von 200 bis 700 °C calciniert wird.

**8.** Verfahren nach Anspruch 7, dadurch gekennzeichnet, daß der calcinierte Katalysator mit Wasserstoffperoxid behandelt und anschließend abfiltriert und getrocknet wird.

**9.** Verwendung eines Katalysators, der nach einem der Ansprüche 1 bis 8 hergestellt wurde, für die Epoxidierung eines Olefins mit Wasserstoffperoxid oder einem organischen Hydroperoxid.

**Claims**

**1.** Process for obtaining a solid catalyst based on amorphous particulate silica comprising titanium (IV) and showing an absorption band at approximately 240 nm in ultraviolet reflection spectrography, with a ratio r of the intensity of UV absorption measured at 240 nm to the intensity of absorption measured at 330 nm equal to or higher than 3, characterized in that it comprises the preparation of a reaction mixture containing a non anhydrous liquid, an initial amorphous particulate silica and $TiF_3$ or $TiF_4$ in a quantity of 0.1 to 10 % by weight of titanium (Ti) relative to the weight of the initial silica and in that this mixture is heated to a chosen temperature from 20°C to the reflux temperature of the said liquid, at atmospheric pressure, and is allowed to react until the initial silica has absorbed a chosen quantity of titanium of between 0.1 and 10 %, in order thus to give the solid catalyst in suspension in the liquid.

**2.** Process according to claim 1, characterized in that no HCl or HF is added to the mixture.

**3.** Process according to claim 1 or 2, characterized in that the liquid is water.

**4.** Process according to claim 1 or 2, characterized in that the liquid is an organic compound which is inert to the action of $TiF_3$ or of $TiF_4$.

**5.** Process according to one of claims 1 to 4, characterized in that the said temperature is from 20°C to approximately 200°C.

**6.** Process according to one of claims 1 to 5, characterized in that the catalyst in suspension in the liquid is filtered off and then dried.

**7.** Process according to claim 6, characterized in that the dried catalyst is calcined at a temperature of 200°C to 700°C.

**8.** Process according to claim 7, characterized in that the calcined catalyst is treated with hydrogen peroxide and then filtered off and dried.

**9.** Use of a catalyst according to one of claims 1 to 8 for epoxidizing an olefin with hydrogen peroxide or an organic hydroperoxide.